# NEUE EUROPÄISCHE PATENTSCHRIFT

(11) **EP 2 896 342 B2**
(45) Veröffentlichungstag und Bekanntmachung des Hinweises auf die Entscheidung über den Einspruch: **21.08.2019**
(45) Hinweis auf die Patenterteilung: 06.04.2016
(21) Anmeldenummer: 15151601.0
(22) Anmeldetag: 19.01.2015
(51) Int. Cl.: A61B 90/70, A47L 15/50, A47L 15/16, A47L 15/42

(54) **Verteilerleiste**
Distribution strip
Réglette de répartition

(30) Priorität: 21.01.2014 DE 102014100635
(43) Veröffentlichungstag der Anmeldung: 22.07.2015
(73) Patentinhaber: Miele & Cie. KG, 33332 Gütersloh (DE)
(72) Erfinder: Ziehn, Andreas, 33813 Oerlinghausen (DE); Mracek, Maik, 33334 Gütersloh (DE); Penner, Roman, 33613 Bielefeld (DE); Schitov, Dennis, 33818 Leopoldshöhe (DE); Sawadsky, Sergej, 33332 Gütersloh (DE)

(56) Entgegenhaltungen:
- CH-A- 506 283
- DE-A1- 3 621 248
- DE-A1- 3 842 436
- DE-A1- 19 847 151
- DE-A1-102008 052 796
- DE-A1-102010 049 684
- DE-B3-102004 029 970
- DE-B3-102004 060 289
- Internet Veröffentlichung 3mach®-Spülprodukte, June 2010 (2010-06), page 3,
- KUNDENPROSPEKTMATERIAL 3MACH®-SPÜLPRODUKTE, June 2009 (2009-06), page 2,
- LIEFERSCHEIN 090609-2 (LIEFERUNG VON FILTERHÜLSE EINGEBAUT IN SPÜLLEISTE)
- BEIPACKZETTEL3MACH®-FILTERHÜLSE
- TECHNISCH UMGESETZTES KONZEPT VON 3MACH®-PRODUKTEN IN EINEM MIELE-RDG, 2008, Heidelberg
- NEUE QUALIFIZIERUNGSPARAMETER BEI DER INSTRUMENTENAUFBEREITUNG, 2011,
- MIC-KORBSYSTEM ALS EINSCHUBWAGEN IN REINIGUNGSMASCHINEN MIT INTEGRIERBAREN FILTERHÜLSEN

## Beschreibung

Die Erfindung betrifft eine Verteilerleiste zur Anordnung an einen Spülflottenanschluss eines Spülkorbs eines Spülautomaten, insbesondere eines Reinigungs- und/oder Desinfektionsautomaten, mit einem einen Strömungskanal bereitstellenden Verteilerrohr, das eine Mehrzahl von Anschlussstellen zur strömungstechnischen Anbindung von zu reinigenden Spülgütern an den Strömungskanal bereitstellt.

Eine derartige Vorteilerleiste ist beispielsweise aus DE-A-19847151 bekannt.

Spülautomaten im Allgemeinen sowie Reinigungs- und/oder Desinfektionsautomaten im Speziellen sind aus dem Stand der Technik an sich gut bekannt.

Vorbekannte Spülautomaten verfügen über einen Spülbehälter. Dieser stellt seinerseits einen Spülraum bereit. Im bestimmungsgemäßen Verwendungsfall dient der Spülraum der Aufnahme von zu reinigendem Spülgut. Der Spülraum ist über eine Beschickungsöffnung des Spülbehälters zugänglich, die mittels einer zumeist verschwenkbar am Spülbehälter angeordneten Tür fluiddicht verschließbar ist.

Zur Spülgutreinigung kommen im bestimmungsgemäßen Verwendungsfall bevorzugterweise Spülkörbe zum Einsatz. Ein solcher Spülkorb wird im Einsatzfall mit zu reinigendem Spülgut bestückt und anschließend verwenderseitig durch die Beschickungsöffnung in den Spülraum eingeführt. Nach erfolgreich durchgeführter Spülung, das heißt Reinigung und/oder Desinfektion des Spülguts kann der Spülkorb zusammen mit den davon aufgenommenen Spülgütern verwenderseitig dem Spülraum durch die Beschickungsöffnung wieder entnommen werden.

Innerhalb des Spülraums ist eine Sprüheinrichtung angeordnet. Diese dient der Beschickung von Spülgut mit Spülflotte. Die Sprüheinrichtung verfügt zu diesem Zweck über Sprüharme, die bevorzugterweise verdrehbar boden- oder deckenseitig des Spülraums oder an den Spülkörben angeordnet sind. Mittels derartiger Sprüharme kann eine Reinigung der freizugänglichen Außenoberflächen von zu reinigendem Spülgut stattfinden. Bei Spülgütern, die innenliegende Hohlräume aufweisen, wie dies zum Beispiel bei medizinischen Instrumenten insbesondere bei dentalen

Übertragungsinstrumenten oder Instrumenten zum Einsatz in der minimalinvasiven Chirurgie der Fall ist, kann eine hinreichende Reinigung der nicht frei zugänglichen Innenoberflächen durch Sprüharme nicht erreicht werden.

Es sind deshalb sogenannte Verteilerleisten, auch Injektorleisten genannt, bekannt geworden, die über Anschlussstellen zum Anschluss zu reinigender Spülgüter verfügen. Die Verteilerleiste ist ihrerseits an einen Spülflottenanschluss angeschlossen, so dass im bestimmungsgemäßen Verwendungsfall über den Spülflottenanschluss Spülflotte in die Verteilerleiste gelangt, von wo aus dann eine Beschickung der an die Verteilerleiste angeschlossenen Spülgüter mit Spülflotte stattfinden kann. Auf diese Weise wird eine Innenreinigung, das heißt eine Reinigung der von den zu reinigenden Spülgütern bereitgestellten Innenoberflächen erreicht.

Obgleich sich die aus dem Stand der Technik vorbekannten Verteilerleisten im alltäglichen Praxiseinsatz bewährt haben, besteht Verbesserungsbedarf. So hat sich insbesondere als nachteilig herausgestellt, dass es innerhalb der Verteilerleisten zu einem erheblichen Druckabfall hinsichtlich der eingeleiteten Spülflotte kommen kann. In der Konsequenz steht ein nicht mehr hinreichender Spüldruck an den zu reinigenden Spülgütern an, was zu einer nur unzureichenden Reinigung derselben führen kann. Dabei ist der sich bei vorbekannten Verteilerleisten im Anwendungsfall einstellende Druckverlust insbesondere durch die anschlussstellenseitig vorgesehen Filter bedingt. Derartiger Filter bedarf es, um zu vermeiden, dass es aufgrund von in der Spülflotte mitgeführten Verschmutzungen zu Verstopfungen in den unter Umständen englumigen Kanälen und/oder Hohlräumen der zu reinigenden Spülgüter kommt. Aus dem Stand der Technik bekannte Filter sind als sogenannte Filterplättchen ausgebildet und aus einem keramischen Material gebildet. Dabei ist je Anschlussstelle der Verteilerleiste ein solcher Filter vorgesehen.

Ausgehend vom Vorbeschriebenen ist es die **Aufgabe** der Erfindung, die aus dem Stand der Technik vorbekannte Verteilerleiste dahingehend weiterzuentwickeln, dass insbesondere das Reinigungs- und/oder Desinfektionsergebnis beeinträchtigende Druckverluste innerhalb der Verteilerleiste weitestgehend vermieden werden.

Zur **Lösung** dieser Aufgabe wird mit der Erfindung eine Verteilerleiste mit den Merkmalen von Anspruch 1 vorgeschlagen.

In Abkehr zur Ausgestaltung nach dem Stand der Technik wird mit der Erfindung anstelle eines separaten Einzelfilters je Anschlussstelle ein für sämtliche Anschlussstellen gemeinsamer Filter vorgeschlagen. Diese Ausgestaltung hat den Vorteil, dass quasi kein Druckverlust eintritt und dass eine im Vergleich zu Einzelfiltern je Anschlussstelle größer Filterfläche zur Verfügung gestellt ist. Dies führt im Ergebnis in vorteilhafter Weise zu einer verbesserten Reinigungswirkung, da die Spülflotte mit einem im Vergleich zum Stand der Technik höheren Druck und damit mit höherer Reinigungswirkung durch die zu reinigenden Spülgüter hindurchgeführt werden kann.

Der nach der Erfindung vorgesehene Filter ist als Siebzylinder ausgebildet, d.h. hat die Form eines Zylindermantels, und ist in dem vom Verteilerrohr bereitgestellten Strömungskanal angeordnet. Im bestimmungsgemäßen Verwendungsfall strömt Spülflotte eingangsseitig, d.h. am proximalen Ende des Verteilerrohres, in das Verteilerrohr ein, wobei sie in den vom Siebzylinder bereitgestellten Hohlraum einströmt. Die Spülflotte durchströmt die Siebzylinderwandung alsdann radial, infolgedessen es zur Spülflottenfilterung kommt. Die so gefilterte Spülflotte gelangt sodann zu den einzelnen Anschlussstellen des Verteilerrohrs, von wo aus dann eine Beschickung der an die jeweiligen Anschlussstellen angeschlossenen Spülgüter stattfindet.

Die vorbeschriebene Konstruktion gestattet in vorteilhafter Weise eine Rückspülung zum Zwecke der Reinigung des Filters. Bei den aus dem Stand der Technik vorbekannten Filterplättchen ist dies nicht möglich. Im Unterschied zum Stand der Technik gestattet die erfindungsgemäße Ausgestaltung mithin eine Art Selbstreinigung durch Spülung bzw. Rückspülung des Filters, was die Einsatzdauer verlängert, so dass insoweit eine vereinfachte Handhabung gegeben ist. Hierzu kann das Verteilerrohr etwa eine, insbesondere am distalen Ende, also an der distalen Stirnseite bzw. Grundfläche des Verteilerrohres angeordnete Sollleckage aufweisen, so dass vom Filter aufgehaltene Schmutzpartikel im normalen Spülprozess kontinuierlich durch die Sollleckage hindurch aus dem Verteilerrohr abgeführt werden. Alternativ umfasst das Verteilerrohr zu dem selben Zweck einen, insbesondere am distalen Ende, also an der distalen Stirnseite des Verteilerrohres angeordneten schaltbaren Ausgang, der manuell und/oder automatisch von einer geschlossenen, insbesondere fluiddichten Stellung in eine geöffnete Stellung und umgekehrt überführt werden kann. Bei einer ersten Betriebsweise oder Normalbetrieb des Spülautomaten befindet sich dieser Ausgang in geschlossener Stellung, wobei die Spülflotte ausschließlich durch die Anschlussstellen aus dem Verteilerrohr entweichen kann, so dass das Spülgut mit vergleichsweise hohem Druck gereinigt wird. Bei einer zweiten Betriebsweise oder Filterreinigungsbetrieb befindet sich dieser Ausgang in geöffneter Stellung, wobei die Spülflotte ungefiltert durch jenen Ausgang aus dem Verteilerrohr entweichen und dabei die innere Filterfläche von Verschmutzungen freispülen kann. Insbesondere kann in einem Spülprogramm ein Programmabschnitt zur Reinigung des Filters vorgesehen sein, bei dem automatisch eine Öffnung des Ausgangs durch eine Steuereinheit des Spülautomaten vorgenommen wird.

Hinzukommt, dass ein Filterwechsel im Unterschied zum Stand der Technik in deutlich einfacherer Weise durchgeführt werden kann. So ist es insbesondere nicht erforderlich, je Anschlussstelle einen Filterwechsel vorzunehmen, so dass auch insoweit die Handhabung mit der erfindungsgemäßen Ausgestaltung verbessert ist. Der Filter, d.h. der Siebzylinder ist, leicht durch das Bedienpersonal entnehmbar, in dem vom Verteilerrohr bereitgestellten Strömungskanal angeordnet.

Ein weiteres kommt hinzu: Zur Anordnung der aus dem Stand der Technik bekannten Filter bedarf es je Anschlussstelle eines Adapterelements, das den eigentlichen Filter aufnimmt. Diese Adapterelemente haben einen vergleichsweise großen Platzbedarf, was in der Konsequenz dazu führt, dass nicht sämtliche Anschlussstellen der aus dem Stand der Technik vorbekannten Verteilerleisten eingeschränkt zugänglich sind. Die Anzahl der je Verteilerleiste aufbereitbaren Spülgüter ist deshalb vergleichsweise niedrig. Die erfindungsgemäße Ausgestaltung schafft hier Abhilfe, da der Einsatz von aus dem Stand der Technik vorbekannten Adapterelementen zur Filterflächenanordnung nicht weiter erforderlich sind. Dies gestattet eine optimiertere Platzausnutzung und damit auch einen optimierteren Einsatz der Verteilerleiste. Es können insbesondere je Verteilerleiste mehr zu reinigende Spülgüter an die Verteilerleiste angeschlossen werden.

Mit der erfindungsgemäßen Ausgestaltung werden insgesamt folgende Vorteile erreicht:
- Aufgrund des gemeinsamen Filters wird eine signifikante Optimierung der Druckverhältnisse in den Wasserwegen erreicht.
- Die insgesamt zur Verfügung stehende Filterfläche wird vergrößert, und zudem ist eine Selbstreinigung durch Rückspülung möglich, was im Unterschied zum Stand der Technik einen mehrmaligen Filterwechsel je Monat erspart.
- Die Bestückung der Körbe kann verwenderseitig flexibler gehandhabt werden, da auf platzeinnehmende Adapterelemente vollends verzichtet werden kann bzw. verzichtet ist.
- Es ist eine einfache und weniger aufwendige Montage sichergestellt, und zwar sowohl werks- als auch kundenseitig, da je Verteilerleiste und nicht mehr je Anschlussstelle eine Filterwartung und/oder ein Filterwechsel vorzunehmen ist.

Gemäß einem weiteren Merkmal der Erfindung ist vorgesehen, dass das Verteilerrohr im Querschnitt kreisförmig ausgebildet ist. Es wird so eine optimierte Durchströmung des vom Verteilerrohr bereitgestellten Strömungskanals erreicht. Insbesondere Strömungstodzonen können so vermieden werden. Ungewollte Schmutz- oder Partikelablagerungen im Strömungskanal können so vermieden werden.

Der Siebzylinder ist gemäß einem weiteren Merkmal der Erfindung aus einem Siebgewebe gebildet. Es kommt bevorzugterweise ein Siebgewebe zum Einsatz, das aus einzelnen übereinander angeordneten Gewebelagen, insbesondere aus Edelstahl, gebildet ist, insbesondere handelt es sich um ein Medium aus sog. Plymesh. Die Maschenweiten des Siebgewebes betragen 100 µm oder weniger, vorzugsweise 80 µm oder weniger, noch mehr bevorzugt 60 µm oder weniger.

Mit der Erfindung wird ferner ein Spülautomat vorgeschlagen, der mit einer Verteilerleiste der vorbeschriebenen Art ausgerüstet ist. Zu diesem Zweck ist vorgesehen, dass der Spülautomat spülrauminnenseitig über Anschlussstutzen für Spülflotte verfügt. Die im bestimmungsgemäßen Verwendungsfall vom Spülraum aufgenommenen Spülkörbe des Spülautomaten verfügen ihrerseits über zu den Anschlussstutzen des Automaten korrespondierende Rohrstücke. Im bestimmungsgemäßen Verwendungsfall greifen diese Rohrstücke in entsprechende Anschlussstutzen ein, so dass Spülflotte zu den einzelnen Körben des Spülautomaten gelangen kann. Die Rohrstücke der Körbe stellen ihrerseits Spülflottenanschlüsse bereit, an welche im bestimmungsgemäßen Verwendungsfall erfindungsgemäße Verteilerleisten angeschlossen sind.

Weitere Vorteile und Merkmale der Erfindung ergeben sich aus der nachfolgenden Beschreibung anhand der Figuren. Dabei zeigen:
- Fig. 1: in explosionsartiger Darstellung eine Verteilerleiste nach der Erfindung und
- Fig. 2: in schematischer Darstellung ein mit einer Verteilerleiste nach der Erfindung ausgerüsteter Spülkorb eines Spülautomaten.

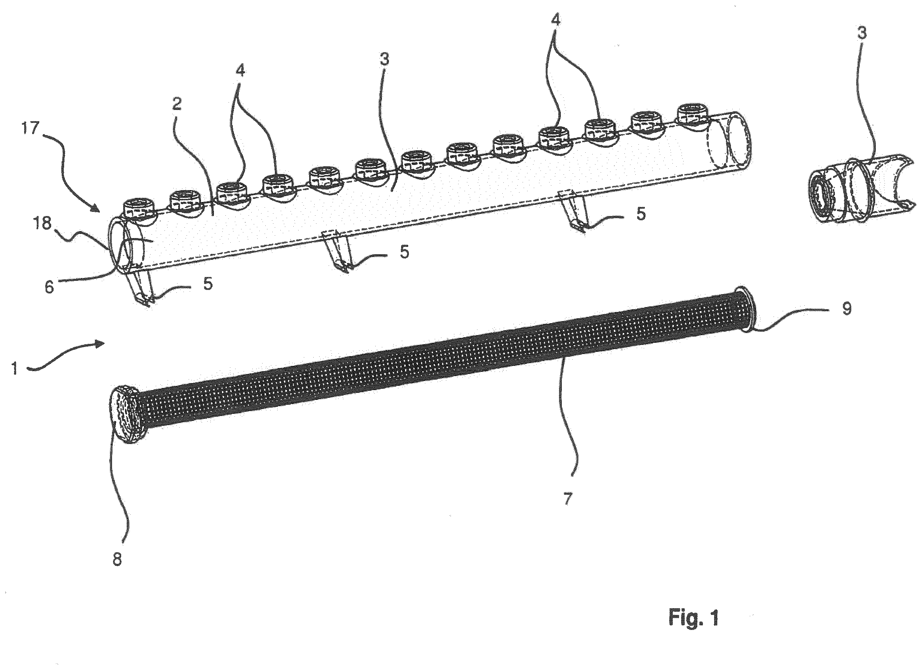

Fig. 1 lässt eine Verteilerleiste 1 beziehungsweise deren Bestandteile erkennen.

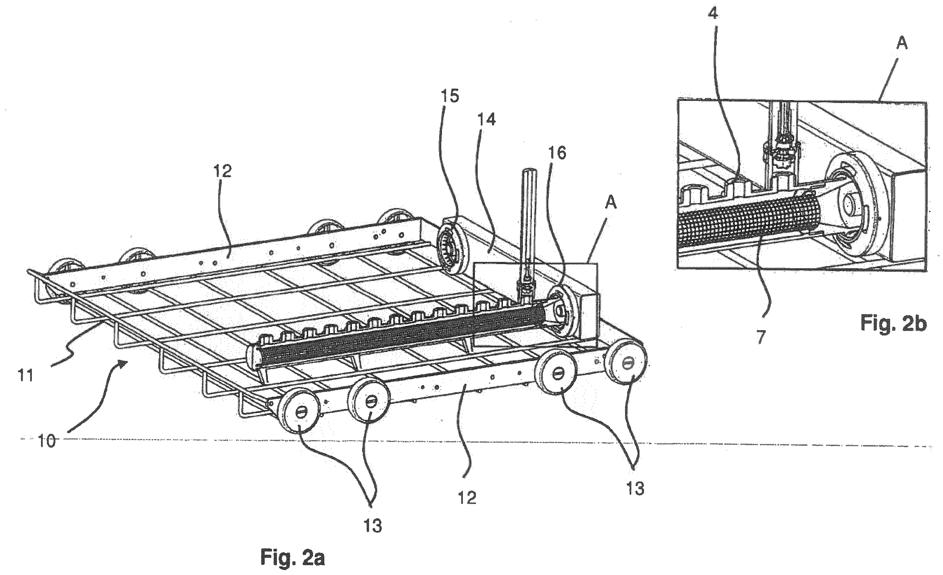

Die Verteilerleiste 1 weist ein Verteilerrohr 2 auf. Dieses ist im Querschnitt kreisförmig ausgebildet und stellt einen Strömungskanal 6 bereit. Zur lagesicheren Positionierung des Verteilerrohrs 2 in beziehungsweise an einem Spülkorb 10 ist das Verteilerrohr 2 mit Stützbeinen 5 ausgerüstet, die sich im endmontierten Zustand am Drahtgeflecht 11 des Spülkorbes 10 abstützen, wie sich dies auch der Darstellung nach Fig. 2 ergibt.

Für den Anschluss zu reinigender Spülgüter, insbesondere medizinischer Instrumente verfügt das Verteilrohr 2 über Anschlussstellen 4. Im bestimmungsgemäßen Verwendungsfall kann an je einer Anschlussstelle 4 ein zu reinigendes Instrument angeordnet sein.

Die Verteilerleiste 1 verfügt erfindungsgemäß über einen für sämtliche Anschlussstellen 4 gemeinsamen Filter, der als Siebzylinder 7 ausgebildet ist. Im endmontierten Zustand ist der Siebzylinder 7 im Strömungskanal 6 angeordnet. Zur endseitigen Abdichtung des Verteilerrohrs 2 an seinem distalen Ende 17 ist eine Verschlusskappe 8 vorgesehen. Diese kann einstückig mit dem Siebzylinder 7 ausgebildet sein. Alternativ kann die Verschlusskappe 8 als Einzelbauteil ausgebildet sein, welches zum Zwecke einer Filterreinigung während einer Betriebsphase weggelassen oder eine Öffnungsstellung gebracht wird. Außerdem kann der mit der Verschlusskappe 8 verschlossene Ausgang 18 auch schaltbar ausgebildet sein und somit von einer Steuereinheit des Spülautomaten veranlasst in eine Öffnungsstellung und umgekehrt überführt werden.

Im Falle eines Filterwechsels kann der Siebzylinder 7 nach einem Lösen der Verschlusskappe 8 in einfacher Weise aus dem Strömungskanal 6 des Verteilerrohrs 2 hinausgeführt und durch einen neuen Siebzylinder 7 ersetzt werden.

Der Siebzylinder 7 ist an seinem der Verschlusskappe 8 gegenüberliegenden Ende mit einer Dichtung 9 ausgestattet. Diese wirkt im endmontierten Zustand dichtend mit dem Verteilerrohr 2 und/oder einem Anschlussadapter 3 zusammen, welcher Anschlussadapter 3 dazu dient, das Verteilerrohr 2 zwecks Versorgung mit Spülflotte an ein spülkorbseitiges Rohrstück 14 anzuschließen, wie dies insbesondere die Darstellung nach Fig. 2 erkennen lässt.

Fig. 2a sowie Fig. 2b, worin das Detail A vergrößert dargestellt ist, lassen einen Spülkorb 10 mit davon aufgenommener Verteilerleiste erkennen. Der Spülkorb 10 ist aus einem Drahtgeflecht 11 gebildet, das mit Bezug auf die Zeichnungsebene nach Fig. 2a links- wie rechtsseitig Leisten 12 aufnimmt, die ihrerseits Rollen 13 tragen. Mittels dieser Rollen 13 kann der Spülkorb 10 in einfacher Weise aus einem von einem in den Figuren nicht näher dargestellten Spülautomaten bereitgestellten Spülraum heraus verfahren beziehungsweise in diesen hinein verfahren werden.

Der Spülkorb 10 ist mit einem Rohrstück 14 ausgestattet. Bei einem bestimmungsgemäß in den Spülraum eines Spülautomaten eingebrachten Spülkorb 10 wirkt dieses Rohrstück 14 mit einer automatenseitigen Spülflottenversorgung zusammen. Im Verwendungsfall strömt also Spülflotte über den automatenseitigen Anschluss in das Rohrstück 14 des Spülkorbs 10 ein.

Das Rohrstück 14 stellt im gezeigten Ausführungsbeispiel zwei Spülflottenanschlüsse 15 und 16 bereit. Diese dienen dem Anschluss einer jeweiligen Verteilerleiste 1, gegebenenfalls unter Zwischenordnung eines Anschlussadapters 3.

### Bezugszeichen

- 1: Verteilerleiste
- 2: Verteilerrohr
- 3: Anschlussadapter
- 4: Anschlussstelle
- 5: Stützbein
- 6: Strömungskanal
- 7: Siebzylinder
- 8: Verschlusskappe
- 9: Dichtung
- 10: Spülkorb
- 11: Drahtgeflecht
- 12: Leiste
- 13: Rolle
- 14: Rohrstück
- 15: Spülflottenanschluss
- 16: Spülflottenanschluss
- 17: Distales Ende des Verteilerrohres
- 18: Ausgang

## Patentansprüche

1. Verteilerleiste zur Anordnung an einem Spülflottenanschluss (15, 16) eines Spülkorbs (10) eines Spülautomaten, insbesondere eines Reinigungs- und/oder Desinfektionsautomaten, mit einem einen Strömungskanal (6) bereitstellenden Verteilerrohr (2), das eine Mehrzahl von Anschlussstellen (4) zur strömungstechnischen Anbindung von zu reinigenden Spülgütern an den Strömungskanal (6) bereitstellt, und mit einem für sämtliche Anschlussstellen (4) gemeinsamen Filter, der als Siebzylinder (7) ausgebildet und in dem vom Verteilerrohr (2) bereitgestellten Strömungskanal (6) angeordnet ist,
**dadurch gekennzeichnet,**
**dass** das Verteilerrohr (2) einen Ausgang (18) aufweist, durch den die Spülflotte ungefiltert aus dem Verteilerrohr (2) strömen kann.

2. Verteilerleiste nach Anspruch 1, **dadurch gekennzeichnet, dass** das Verteilerrohr (2) im Querschnitt kreisförmig ausgebildet ist.

3. Verteilerleiste nach Anspruch 1 oder 2, **dadurch gekennzeichnet, dass** der Siebzylinder (7) aus einem Siebgewebe, vorzugsweise Plymesh gebildet ist.

4. Verteilerleiste nach Anspruch 3, **dadurch gekennzeichnet, dass** die Maschenweite des Siebgewebes 100 µm, vorzugsweise 80 µm, noch mehr bevorzugt 60 µm, oder kleiner beträgt.

5. Verteilerleiste nach einem der vorhergehenden Ansprüche, **dadurch gekennzeichnet, dass** der Ausgang (18) am distalen Ende (17) des Verteilerrohres (2) angeordnet ist.

6. Verteilerleiste nach einem der vorhergehenden Ansprüche, **dadurch gekennzeichnet, dass** der Ausgang (18) zwischen einer geöffneten und einer geschlossenen Stellung schaltbar ausgebildet ist.

7. Spülautomat, insbesondere Reinigungs- und/oder Desinfektionsautomat, ausgerüstet mit einer Verteilerleiste (1) nach einem der vorhergehenden Ansprüche 1 bis 6.

## Claims

1. Manifold bar for arrangement on a rinsing solution connection (15, 16) of a rinsing rack (10) of an automatic rinsing machine, in particular a cleaning and/or disinfection machine, comprising a manifold pipe (2) which provides a flow channel (6) and provides a plurality of connection points (4) for washware to be rinsed to be fluidically connected to the flow channel (6), and comprising a filter which is shared by all the connection points (4), is in the form of a perforated cylinder (7) and is arranged in the flow channel (6) provided by the manifold pipe (2),
**characterised in that** the manifold pipe (2) comprises an outlet (18) through which the rinsing solution can flow, unfiltered, out of the manifold pipe (2).

2. Manifold bar according to claim 1, **characterised in that** the manifold pipe (2) has a circular cross section.

3. Manifold bar according to either claim 1 or claim 2, **characterised in that** the perforated cylinder (7) is formed from a screen mesh, preferably Plymesh.

4. Manifold bar according to claim 3, **characterised in that** the mesh size of the screen mesh is 100 µm, preferably 80 µm, even more preferably 60 µm, or smaller.

5. Manifold bar according to any of the preceding claims, **characterised in that** the outlet (18) is arranged at the distal end (17) of the manifold pipe (2).

6. Manifold bar according to any of the preceding claims, **characterised in that** the outlet (18) is formed so as to be switchable between an open and a closed position.

7. Automatic rinsing machine, in particular a cleaning and/or disinfection machine, equipped with a manifold bar (1) according to any of the preceding claims 1 to 6.

## Revendications

1. Barrette de distribution destinée à être disposée sur un raccord (15, 16) de solution de lavage d'un panier de lavage (10) d'un appareil de lavage automatique, en particulier d'un appareil automatique de nettoyage et/ou de désinfection, avec un tube de distribution (2), réalisant un conduit d'écoulement (6), qui réalise une pluralité de points de raccordement (4) pour relier en écoulement des articles à laver destinés à être nettoyés avec le conduit d'écoulement (6), et avec un filtre, commun à tous les points de raccordement (4), qui est constitué en tant que cylindre de tamisage (7) et qui est disposé dans le conduit d'écoulement (6) réalisé par le tube de distribution (2),
**caractérisée en ce que** le tube de distribution (2) présente une sortie (18) à travers laquelle la solution de lavage peut quitter le tube de distribution (2) sans être filtré.

2. Barrette de distribution selon la revendication 1, **caractérisée en ce que** le tube de distribution (2) est constitué avec une section transversale de forme circulaire.

3. Barrette de distribution selon la revendication 1 ou 2, **caractérisée en ce que** le cylindre de tamisage (7) est formé d'un tissu de tamisage, de préférence en Plymesh.

4. Barrette de distribution selon la revendication 3, **caractérisée en ce que** la largeur de maille du tissu de tamisage est égale à 100 µm, de préférence 80 µm, de façon encore plus préférée 60 µm, ou est plus petite.

5. Barrette de distribution selon l'une des revendications précédentes, **caractérisée en ce que** la sortie (18) est disposée à l'extrémité distale (17) du tube de distribution (2).

6. Barrette de distribution selon l'une des revendications précédentes, **caractérisée en ce que** la sortie (18) est constituée de façon commutable entre une position ouverte et une position fermée.

7. Appareil de lavage automatique, en particulier un appareil automatique de nettoyage et/ou de désinfection, équipé d'une barrette de distribution (1) selon l'une des revendications précédentes 1 à 6.
